Europäisches Patentamt

European Patent Office

Office européen des brevets

(19)

(11) Publication number: 0 335 714
A2

# (12) EUROPEAN PATENT APPLICATION

(21) Application number: 89303149.2

(22) Date of filing: 30.03.89

(51) Int. Cl.⁴: A 61 B 17/22

(30) Priority: 31.03.88 US 176586

(43) Date of publication of application:
04.10.89 Bulletin 89/40

(84) Designated Contracting States:
BE DE ES FR GB IT LU NL

(71) Applicant: SITE MICROSURGICAL SYSTEMS, INC.
135 Gibraltar Road
Horsham Pennsylvania 19044 (US)

(72) Inventor: Krauss, Ray
87 South Wiggins Lane
Skillman, NJ 08558 (US)

Rockley, Paul W.
45 Laurel Circle
Newton, PA 18940 (US)

(74) Representative: Mercer, Christopher Paul et al
Carpmaels & Ransford 43, Bloomsbury Square
London WC1A 2RA (GB)

(54) Means for tissue removal using laser technology.

(57) A handpiece which is capable of performing cataract removal within the eye. A fiber optic lead is provided to cooperate with irrigation and aspiration means within the handpiece. Insertion means containing connections between the fiber optic lead and an optical window are attached to the handpiece for placement near cataractus lens tissue, after a microscopic cut has been made on the eye surface. The insertion means is held by the handpiece, and is also connected to irrigation and aspiration leads. The fiber optic member is connected to a light source capable of tissue ablation, usually an Erbium: YAG laser.

FIG-1

**Description**

## MEANS FOR TISSUE REMOVAL USING LASER TECHNOLOGY

### FIELD OF THE INVENTION

This invention relates generally to means for removal of tissue. Specifically, this invention relates to the removal of tissue using lasers. More specifically, this invention relates to the ablation of ocular tissue using laser technology.

### BACKGROUND OF INVENTION

It is well known that there are many different possible means for removal of ocular tissue, especially removal of hardened ocular cataract tissue. One of the more common methods of cataract removal is intracapsular removal, originally performed in the late nineteenth century. In addition, some of the more recent preferred methods of capsular removal have been by means of the process known as phacoemulsification, in which the simultaneous irrigation and aspiration of the eye tissue is performed in the lens capsule. The cataractus lens tissue is broken by a needle vibrating at ultrasonic frequencies, usually in the range of about 27-55 kHz. Continuous irrigation of the tissue is provided to replace removed fluid and to create a slurry composed of the irrigation fluid and the emulsified lens tissue. The tissue is removed through the suction process known as aspiration. A new intraocular lens is then placed in the evacuated chamber.

The phacoemulsification-type surgery requires some form of knife cut into the cornea, by which the instruments for irrigation and aspiration can be emplaced near the cataract. Generally, these cuts must be at least 6 mm in length for proper insertion of the intraocular replacement lenses and freedom of movement of the ultrasonic equipment.

This method, while extremely useful, has certain limiting drawbacks. First, the technique of phacoemulsification can be tedious to perform, as it necessitates steady hand-to-eye coordination for the doctor performing the process. Second, certain phacoemulsification equipment can pose heat transfer difficulties. In other words, the eye tissue near the cataractus tissue must be maintained at lower temperatures than the temperature generated by the oscillatory motion of the ultrasonic needle. Third, the relatively enlarged cut made in the eye in order to accommodate intraocular lens implants will, of course, be more obtrusive than any possible smaller cuts.

Naturally, with the recent advances in the field of laser technology, heightened scientific activity has turned toward lasers as possible means for performing cataract surgery. However, many parameters must be met to successfully use lasers in this type of surgery. To date these parameters have not been reached. Among these requirements are: (1) A laser capable of removing all the cataractus lens material; (2) A laser capable of nearly virtual contact with the tissue to be removed, by which only a small volume of tissue will be removed; (3) A fiber optic piece which can conduct the laser to the eye for proximate use to ablate the cataract or other tissue; (4) A laser capable of use within the eye; that is, a laser which will not generate thermal effects causing it to ablate other parts of the eye; (5) A laser or fiber optic handpiece capable of insertion within a very small (less than 3 mm) cut in the corneal tissue of the eye; and (6) A laser capable of being maintained for use within a manageable handpiece.

### SUMMARY OF THE INVENTION

Therefore, it is an object of the present invention to provide a surgical device capable of removing cataractus eye tissue or any other material chosen to be photoablated. It is a further object to the invention to provide a laser device capable of fragmenting only cataract material (of about 1.0-1.5 microns in depth) away from the tip of the fiber optic piece, and not other eye tissue near the cataract.

It is yet another object of this invention to provide a laser which is capable of being inserted through a small incision (less than 3 mm) into the eye.

It is still a further object of the present invention to provide a laser capable of placement within an operating handpiece for use by a surgeon.

An additional object of the present invention is to provide a laser in which the optical element is capable of contact with the cataractus lens tissue to be removed.

It is still another object of this invention to provide a laser attachable to a relatively small power supply pack, which will provide irrigation and aspiration sources as well as the light source necessary to direct the laser used to perform the surgery on the tissue to be ablated.

These and other objects of the present invention are achieved by means of a laser device in which the wavelength of light used is in the range of about 2.94 microns, commonly called the Erbium: YAG Laser. This laser is attached to a monofilament fiber optic manufactured from zirconium fluoride or some similar material, and is transmitted through a synthetic sapphire window upon the eye tissue to be removed. The energy in the laser will absorb all of the water in the cataractus lens tissue and, therefore, remove the tissue from its hold within the lens capsule.

In the tubular means which carry the fiber optic element, there are also provided irrigation means to provide the necessary lubricant to the emulsified tissue and aspiration means to remove the particulate created during the laser photoablation process. The total length of the necessary incision into the cornea will be roughly 1.5-3.0 mm, or potentially less than one half the minimum 3.2 mm commonly used to accomodate intraocular lens implants during phacoemulsification.

The fiber optic monofilament cable, as well as the irrigation means and aspiration means, are attached to a handpiece. This handpiece can further be attached to a power supply to provide the necessary pumping sources and laser energy in order to perform the photoablation procedure. Because the handpiece is small and the necessary power supply manageable for hand carrying, the present invention is usable in a portable manner, such that it can be taken from surgery room to surgery room.

In addition, the fiber optic means, irrigation means and aspiration means are generally removable for replacement and disposal. Connection means are provided for both the irrigation and aspiration ports as well as the fiber optic ports. It is this feature which allows the user to connect or disconnect the fiber optics to transport the handpiece, and allow the handpiece to be used over and over again on different patients, or to be disposable, if desired.

Other objects, advantages and features of the present invention are apparent from the following detailed description of the drawings, taken in conjunction with the following detailed description of the invention, to disclose a preferred embodiment of the present invention, in which:

DETAILED DESCRIPTION OF THE DRAWINGS

Fig. 1 is a perspective view of a handpiece containing the fiber optic element used in the laser of the present invention;

Fig. 2 is a top cross-section of a handpiece made in accordance with a preferred embodiment of the present invention, along lines 2-2 of Fig. 1;

Fig. 3 is a top view of the end of a fiber optic element as disclosed in accordance with a preferred embodiment of the present invention; and

Fig. 4 is a side view of a fiber optic element for insertion in a handpiece of the present invention.

DETAILED DESCRIPTION OF THE INVENTION

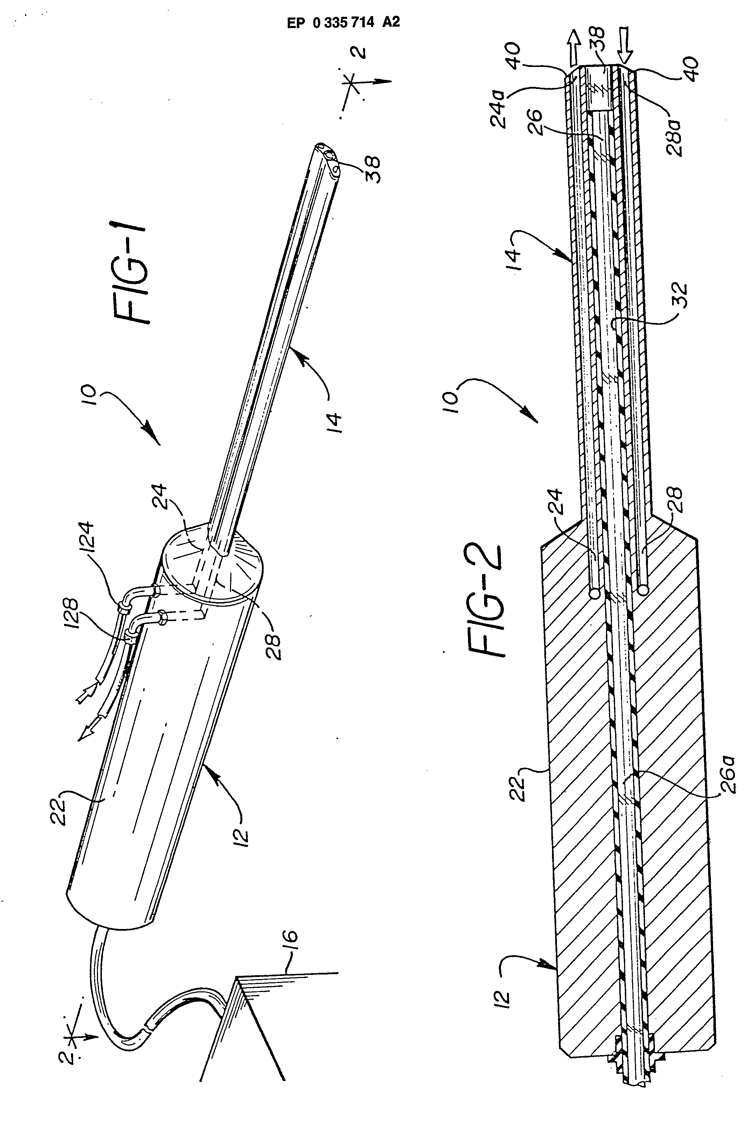

Referring now to Fig. 1, the laser 10 of the present invention is comprised of handpiece 12 attached to tubular fiber optic insertion means 14. It is generally the handpiece 12 which a surgeon can hold freely in order to effectively perform the operation. It is generally the fiber optic insertion means 14 which is placed within the eye for photoablation of the cataractus lens tissue.

The handpiece 12 is also attached to a laser energy or power supply 16 which provides the laser energy to be used during the operation of the present invention. The handpiece 12 is also attached to an irrigation source 18 and an aspiration pump 20, both of which will provide the necessary lubricant and suction around the eye tissue that is about to be ablated.

Referring now to Fig. 2, in the handpiece barrel 22 are contained irrigation or infusion lead 24, fiber optic lead 26, and aspiration lead 28. These leads 24, 26, 28 are bored along the center of the handpiece barrel 22, and continue at the point of attachment of the handpiece barrel 22 and the fiber optic insertion means 14. When guided into the fiber optic insertion means 14, the infusion lead 22 and the aspiration lead 24 generally each have a diameter between about .013 and .025 inches, or between 0.5 and 1.0 mm. The fiber optic piece itself generally has a diameter of about .005-.015 inches, or between about 200 microns to about 500 microns (0.2 mm-0.5 mm).

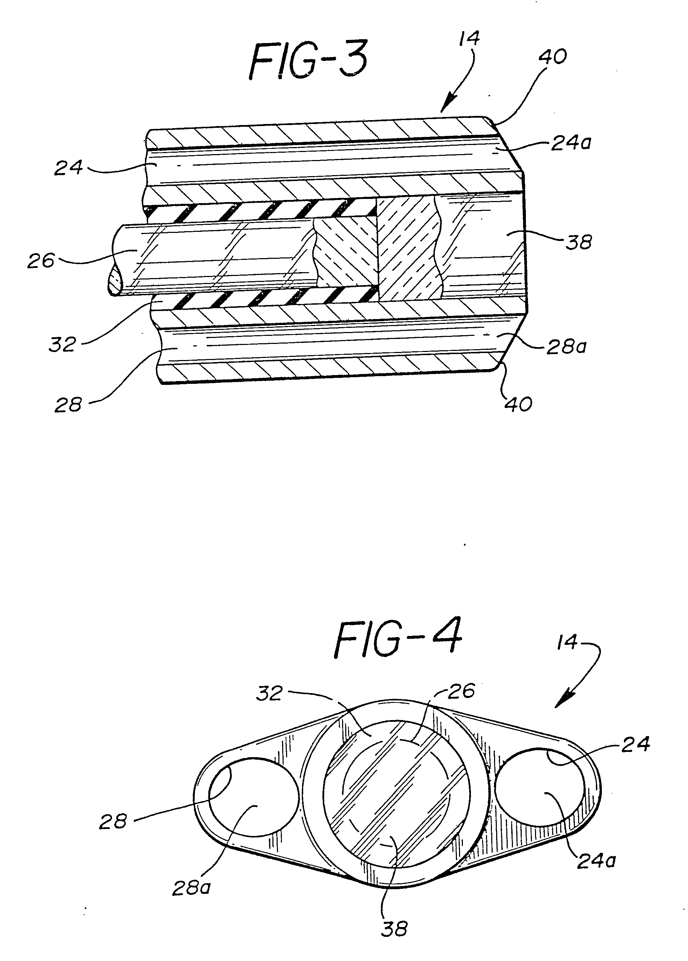

As seen in Figs. 3 and 4, the fiber optic lead 26 is generally a 1 mm barrel (19 gauge) tube, which fits in the handpiece 22, which also contains infusion lead 24, and aspiration lead 28. As seen Fig. 4, the height of the fiber optic insertion means 14 as described is generally held within .027 inches, with a width between .0306 and .0330 inches, or roughly 1.0-1.2 mm. It is this fiber optic lead 26 which will be inserted into the eye at the end of insertion means 14. The fiber optic lead 26 is cladded with material 32 to enable the laser beam to reflect along the length of the fiber optic lead 26. This cladding material 32, generally formed an acrylate, becomes more readily apparent in Fig. 3. The fiber optic lead 26 may also be jacketed with a protective hardening coating. The fiber optic lead 26 is press fit between the infusion lead 24 and aspiration lead 28, at the center of the fiber optic insertion means 14. The fiber optic lead 26 ends at an optical window insertion port. This insertion port enables the fiber optic lead 26 to be attached to a corresponding optical window attachment 38, generally made of synthetic sapphire. Any material harder than the optical fiber and capable of transmitting laser energy is acceptable. When the optical window 38 is attached to the end of the fiber optic lead 26, the laser becomes capable of abutting on the area to be ablated.

Returning to Figs. 1 and 2, generally, the fiber optic insertion means 14 also has an open end which provides an irrigation port 24a, and an aspiration port 28a. At its other end, the fiber optic insertion means 14 is connected to the handpiece 12. These are accomplished by connection means 124, 126 and 128. First connection means 124 is connected to the infusion lead 24. The first connection means is then connected to an irrigation source 18. The second connection means 128 connects the fiber optic lead 26 to the handpiece fiber optic 26a. Handpiece fiber optic 26a is then connected to light source means 16. Third connection means 128 connect the aspiration lead 28 to the aspiration pump 20.

The fiber optic leads 26 and 26a are generally cables of monofilament ziconium fluoride (ZrF₄) or any other suitable material. This fiber was chosen because it is among those best able to conduct the appropriate light wavelength. This light must be a laser capable of ablating the entire water content of the eye tissue without disintegrating the fiber optic leads 26, 26a. Ziconium fluoride generally contains a water content of less than the threshold above which a photoablative laser such as the Erbium: YAG will disintegrate the fiber optic leads 26, 26a. Naturally, other substitutes having the same conducting characteristics as zirconium flouride will suffice to carry the laser energy.

As seen in Figs. 3 and 4, the end of the fiber optic insertion means 14 has an optical window 38 which enables direct contact with the cataract tissue to be ablated. Thus, the laser energy source will be carried from the power supply 16 through the fiber optic leads 26, 26a to the optical window 38 for tissue ablation. As also seen in Fig. 2, the two side leads 24 and 28 make possible irrigation and aspiration at the location of laser ablation.

The choice of a laser was made on the basis of having a light source capable of breaking the hydrogen-oxygen bond in water. This results from the composition of the cataract, which is nearly all water. The laser photons are efficiently absorbed by the water molecules in a cataract. The photons' high excitation level ultimately causes the hydrogen/oxygen bonds in the water to vibrate apart. Excess energy is given off in the form of steam. This excess energy causes the molecules to break down leaving only pulverous cataract eye tissue. These tissue particles are evacuated when the steam is taken away from the photoablation site.

Because it must be capable of absorbing all the water of the tissue at the surface to be ablated, choice of a laser was limited to one having the characteristics of the absorption peaks of water. Thus, one of the more likely choices is the use of an Erbium: YAG (yttrium-aluminum garnet) laser. This light source has a wavelength of about 2.80-3.00 microns, or more precisely about 2.94 microns, which is a wavelength extremely similar to the wavelength for the peak absorption of bonds in water. Of course, other lasers having Erbium as the host, with a number of potential dopants, are possible. Such possibilities include, but are not limited to, Er:YLF, Er:YALO, Er:YSGG, and Er:CaF$_2$.

Because the bonds in water are broken so quickly, it is found that for an Erbium: YAG laser having a pulse width of about 200 microseconds at a pulse rate of about 10 nanoseconds will be enough to peel off about 1.0-1.5 microns of tissue. This is possible when the energy imparted into the tissue is kept at roughly 1.0 joule per square centimeter, well above the threshold energy level of 0.7 joules per square centimeter. At this energy level, only the 1.0-1.5 microns of tissue are destroyed, and not the surrounding tissue. Nevertheless, a laser lensectomy can be accomplished very quickly, because only about 50 total joules of energy are necessary to remove the entire cataract.

A Schwartz Erbium: YAG laser was used in the present embodiment. Of course, any Erbium: YAG laser is possible for use. The fiber optic insertion means 14 was maintained so that only the end of the optical window 38 is capable of touching the surface to be ablated.

The fiber optic insertion means 14 was kept so that it has a diameter between about 1.6-3.2 mm, and a height of about 1.0 mm. This diameter is a substantial improvement over the diameter of the insertion means used in the phacoemulsification processes, and meets the objective of making a corneal incision of less than the minimum 3.2 mm required for insertion of the lens to the laser capsule.

In operation, therefore, the handpiece 12 is connected to the laser energy supply 16 and the irrigation source 18 as well as the aspiration pump 20. A optical window 38 is placed in the optical window insertion port, and is connected to the handpiece barrel 12. The eye is cut with a very small (less than 3 mm) incision for insertion of the laser. After insertion, the laser is operated at the Erbium: YAG 2.94 micron wavelength for 200 microsecond pulses, enough to remove the catarized eye material. During the operation, irrigation and aspiration occur with enough regularity to keep a constantly lubricated surface and as well as a capsule clear of ablation debris. The amount of cataract tissue material removed at each pulse of the laser is roughly about 1.0-1.5 microns depth of material. After about 50 joules of energy are used, the entire cataract is fragmented and removed. Only the cataract material is removed and not the tissue surrounding the lens itself. This is due to the minute distance maintained between the end of the optical window 38 and the tissue to be removed. After the operation, a new optical window 38 and/or an entire new insertion means 14 may be placed on the handpiece 12 for the next operation. On the other hand, the used pieces can be autoclavable for reuse.

Thus, the unique combination of features possessed by the present invention render them suited for in-the-eye cataract surgery. Of course, while several means are available, a particularly advantageous embodiment has been chosen to illustrate the invention. It will be understood by those skilled in the art that various changes and modifications can be made therein without departing from the scope of the invention, which is defined by the following claims and their equivalents.

## Claims

1. Means for removing tissue from the eye comprising:

(a) fiber optic means for transporting pulses of laser energy having a wavelength corresponding to peaks of water absorption, said pulses of laser energy capable of creating fragmented residue from said tissue;

(b) irrigation means for lubricating said residue, said irrigation means further cooling the area surrounding the tissue to be removed;

(c) aspiration means for removing the tissue fragmented by said laser pulses; and

(d) holding means to combine said fiber optic means, said irrigation means and said aspiration means.

2. The means for removing tissue of claim 1 wherein said holding means comprises:

tubular insertion means, said tubular insertion means comprising a tube having two open ends, one of said open ends providing an irrigation port, an aspiration port and a fiber optic port, said second open end adapted for connection to a handpiece, said tube comprising a plurality of chambers for conducting a fiber optic lead, an aspiration lead and an irrigation lead;

a handpiece for connection to said tubular insertion means;

first connection means to connect said irrigation means to an irrigation source;

second connection means to connect said aspiration means to an aspiration source; and

third connection means to connect said fiber optic means to a light source.

3. Means for photoablating tissue from the eye comprising:

(a) fiber optic means for transporting laser energy in the form of pulses, with a wavelength of one of the absorption peaks of water, said laser energy capable of fragmenting the tissue to be removed;

(b) window means for transmission of said laser energy pulses comprising a synthetic sapphire optical window;

(c) irrigation means for providing lubricant to the area surrounding said tissue to be photoablated;

(d) aspiration means for removing said photoablated tissue;

(e) insertion means for grouping said fiber optic means, said window means, said irrigation means and said aspiration means in a tubular bundle, said insertion means insertable into the eye through a corneal incision; and

(f) holding means to manually operate said insertion means near to said eye.

4. The means of photoablating tissue of claim 3 wherein said insertion means comprises:

a tube having two open ends, one of said open ends providing an irrigation port, an aspiration port and a fiber optic port, said second open end adapted for connection to said holding means, said tube further comprising a plurality of chambers for conducting a fiber optic lead, an aspiration lead and an irrigation lead.

5. The means for photoablating tissue of claim 4 wherein said holding means comprises a handpiece for connection to said insertion means, first connection means for connection of said irrigation means to an irrigation source, second connection means to connect said aspiration means to an aspiration source and third connection means to connect said fiber optic means to said light source means.

6. Means for removing tissue from the eye comprising:

(a) fiber optic means for transporting light to the tissue to be removed, said fiber optic means comprising a strand of zirconium fluoride;

(b) light source means for transportation of pulses of laser energy along said fiber optic means, said light source means comprising an Erbium:YAG laser, said light source means connected to said fiber means within said laser;

(c) window means for transmitting said light source means for removal of said tissue, said window means comprising a synthetic sapphire attachment to the end of said fiber optic means;

(d) irrigation means for lubricating said tissue to be removed, said irrigation means adjacent said fiber optic means;

(e) aspiration means for removal of said eye tissue material, said aspiration means adjacent said fiber optic means;

(f) tubular means to hold said fiber optic means, said window means, said irrigation means and said aspiration means in one bundle;

(g) insertion means for placing said tubular means inside a corneal incision in the eye; and

(h) handpiece means for holding said tubular means during removal of said tissue.

7. The means for removing tissue of any one of claims 1 to 6 wherein said fiber optic means further comprises light source means capable of transmitting pulses of laser light having a wavelength in the range of about 2.8-3.0μm.

8. The means for removing tissue of claim 7, wherein said light source means transmits said pulses of light with an energy per unit area of fiber optic means of at least 0.7 joule/cm$^2$.

9. The means for removing tissue of claim 7 or claim 8, wherein said pulses are directed at said tissue at a pulse width of about 200 microseconds.

10. The means for removing tissue of any one of claims 7 to 9, wherein said pulses are directed at said tissue at a pulse rate of about 10 nanoseconds per pulse.

FIG-1

FIG-2

# FIG-3

# FIG-4